# EUROPÄISCHE PATENTSCHRIFT

(11) **EP 3 241 532 B1**
(45) Veröffentlichungstag und Bekanntmachung des Hinweises auf die Patenterteilung: **30.01.2019**
(21) Anmeldenummer: 17169143.9
(22) Anmeldetag: 02.05.2017
(51) Int. Cl.: A61F 5/01, A41D 13/00

(54) **ORTHESE AUS EINEM ELASTISCHEN TEXTILEN MATERIAL**
ORTHOSIS COMPRISING AN ELASTIC TEXTILE MATERIAL
ORTHÈSE CONSTITUÉE D'UN MATÉRIAU SOUPLE ÉLASTIQUE

(30) Priorität: 02.05.2016 DE 102016108151
(43) Veröffentlichungstag der Anmeldung: 08.11.2017
(73) Patentinhaber: Eisert, Claudia, 64367 Mühltal (DE)
(72) Erfinder: Eisert, Claudia, 64367 Mühltal (DE)
(74) Vertreter: Katscher Habermann Patentanwälte

(56) Entgegenhaltungen:
- WO-A1-02/064073
- WO-A1-2012/114209
- DE-A1-102006 021 321
- US-A- 5 819 322
- US-A1- 2013 104 280

## Beschreibung

Die Erfindung betrifft eine Orthese mit einem Rumpfabschnitt und mit mindestens einem Extremitätenabschnitt, wobei der Extremitätenabschnitt aus einem elastischen textilen Material hergestellt ist und bei der bestimmungsgemäßen Verwendung eine Körperextremität eines Benutzers von dem Rumpfabschnitt bis zu einem dem Rumpfabschnitt abgewandten Endbereich des Extremitätenabschnitts mindestens abschnittsweise umhüllt und dabei eine Kompressionswirkung ausübt, und wobei der Extremitätenabschnitt eine sich entlang des Extremitätenabschnitts von dem Rumpfabschnitt bis zu dem gegenüberliegenden Endbereich erstreckende Funktionsnaht aufweist.

Eine solche Orthese gemäß des Oberbegriffs von Anspruch 1 ist aus der US 2013/104280 A bekannt.

Eine Orthese ist ein medizinisches Hilfsmittel, das zur Stabilisierung, Entlastung, Ruhigstellung, Führung oder Korrektur von Gliedmaßen oder des Rumpfes eines Benutzers eingesetzt werden kann. Mit den eingangs genannten Orthesen, die aus einem geeigneten elastischen textilen Material hergestellt sind, kann durch die mit der Orthese erzeugte Kompressionswirkung die Ausrichtung und Stabilität des Bewegungsapparates unterstützt werden. Neben einem sensorischen Feedback wird gezielt Widerstand für den Bewegungsapparat aufgebaut. Gewünschte Bewegungsmuster können dadurch verstärkt werden. Mit der Orthese kann Haltungsfehlern oder Stellungsfehlern sowie fehlerhaft durchgeführten Bewegungsabläufen therapeutisch entgegengewirkt werden und gegebenenfalls eine dauerhafte Verbesserung erreicht werden.

Es sind Orthesen aus einem elastischen textilen Material bekannt, die beispielsweise wie eine Hose, eine Weste oder ein Shirt ausgestaltet sind und von dem Benutzer wie ein entsprechendes Kleidungsstück getragen werden können. Mit den Extremitätenabschnitten können beispielsweise Arme oder Beine des Benutzers umhüllt werden. Mit dem Rumpfabschnitt kann der Extremitätenabschnitt an einem Übergangsbereich zu dem Rumpf festgelegt und fixiert werden. Der Rumpfabschnitt kann einen den Rumpf teilweise oder vollständig umhüllenden Torsoabschnitt aufweisen. Es ist ebenfalls möglich, dass der Rumpfabschnitt ein Band oder mehrere Bänder aufweist, mit denen der Extremitätenabschnitt, beispielsweise ein Ärmel oder ein Hosenbein, zweckmäßigerweise drehfest an dem Rumpf festgelegt werden kann.

Aus der Praxis sind verschiedene Varianten von langarmigen oder kurzarmigen Orthesen bzw. von Orthesen mit langen oder kurzen Beinen bekannt. Um in einzelnen Bereichen der Orthese zusätzlich zu der Kompressionswirkung weitere Kräfte oder Momente auf den Benutzer ausüben zu können ist es aus der Praxis bekannt, in den betreffenden Bereichen Zugbänder bzw. Zügel anzuordnen. Durch die Ausgestaltung und Anordnung der Bänder oder Zügel können in einer vorgegebenen Richtung zusätzliche zu der Kompressionswirkung weitere Kräfte und Momente erzeugt werden, mit welchen beispielsweise eine Rotationswirkung auf die Arme oder Beine oder auf den Rumpf ausgeübt werden kann.

Es ist ebenfalls bekannt, ein Zugband oder einen Funktionsabschnitt aus einem zweiten Material mit elastischen Eigenschaften, die sich von dem elastischen textilen Material der Orthese unterscheiden, in eine daran angepasste Aussparung in den Rumpfabschnitt oder in den Extremitätenabschnitt der Orthese einzusetzen. In den aneinander angrenzenden und miteinander verbundenen Randbereichen des eingesetzten Zugbands bzw. Funktionsabschnitts und des textilen Orthesenmaterials werden durch die unterschiedlichen elastischen Eigenschaften Vorspannungen erzeugt, so dass in Abhängigkeit von der Ausgestaltung und Anordnung der eingesetzten Zugbänder oder Funktionsabschnitte zusätzliche Kräfte und Momente auf eine Extremität ausgeübt werden können.

Die aus der Praxis bekannten Orthesen mit Zugbändern, bzw. Zügeln verursachen in den Verbindungsbereichen der Zugbänder oder Zügel mit den angrenzenden Extremitätenabschnitten oder den Rumpfabschnitten jeweils lokal begrenzte Zugwirkungen, und bei einer geeigneten Ausrichtung und Anordnung der Zügel auch Drehmomente, die lokal ein Verdrehen der Extremität unterstützen oder behindern können. Eine über einen größeren Bereich der Extremität hinweg gleichmäßige Kraftwirkung lässt sich dagegen nur mit mehreren Zugbändern oder Zügeln erreichen.

Um einem ungewollten Verdrehen einer Extremität in deren Ruhestellung, aber auch bei häufig wiederholten Bewegungsabläufen entgegenzuwirken ist es oftmals wünschenswert, dass mit der Orthese eine möglichst vorteilhafte Rotationswirkung auf den Extremitätenabschnitt ausgeübt werden kann. Es wird deshalb als eine Aufgabe der vorliegenden Erfindung angesehen, die Orthese so auszugestalten, dass mit möglichst einfachen Mitteln und kostengünstig zusätzlich zu der Kompressionswirkung eine gerichtete Zugwirkung oder eine Rotationswirkung in dem Bereich des Extremitätenabschnitts ausgeübt werden kann. Die Rotationswirkung soll sich gegebenenfalls möglichst gleichmäßig über einen weiten Bereich des Extremitätenabschnitts erstrecken.

Diese Aufgabe wird erfindungsgemäß durch eine Orthese der eingangs genannten Gattung gelöst, wobei die beiden längs der Funktionsnaht miteinander verbundenen Randbereiche aus dem elastischen textilen Material eine voneinander abweichende Vorspannung aufweisen. Durch die unterschiedliche Vorspannung der beiden miteinander verbundenen Randbereiche entlang der Funktionsnaht entstehen innerhalb des Extremitätenabschnitts Bereiche mit einer unterschiedlichen Kompressionswirkung. Zudem kann durch eine geeignete Vorgabe der jeweiligen Vorspannung entlang der Funktionsnaht eine Formgebung des Extremitätenabschnitts so vorgegeben werden, dass bei der bestimmungsgemäßen Verwendung der Orthese nicht nur von außen nach innen wirkenden Kompressionskräfte, sondern auch seitlich gerichtete Kräfte oder Drehmomente auf die von dem Extremitätenabschnitt umhüllte Extremität ausgeübt werden. Auf diese Weise können ohne zusätzliche Bänder oder Zügel, die gesondert hergestellt und entweder außen an der Orthese angebracht werden oder in die Orthese eingearbeitet werden müssen, eine therapeutische Wirkung der Orthese verbessert sowie ein Tragekomfort erhöht werden. Die erfindungsgemäße Ausgestaltung der Orthese kann sowohl bei vorkonfektionierten Orthesen als auch bei individuell an einen Benutzer angepassten Orthesen vorgesehen sein und eingesetzt werden.

Gemäß einer besonders vorteilhaften Ausgestaltung des Erfindungsgedankens ist vorgesehen, dass der Extremitätenabschnitt aus einem einzigen Materialzuschnitt des elastischen textilen Materials hergestellt ist. Durch die Verwendung eines einzigen Materialzuschnitts wird die Herstellung des Extremitätenabschnitts erheblich vereinfacht. Die Orthese kann ohne nennenswerten zusätzlichen Herstellungsaufwand so ausgestaltet und gegebenenfalls individuell angepasst werden, dass zusätzlich zu der Kompressionswirkung weitere Kräfte bzw. Drehmomente auf die Extremitäten ausgeübt werden können.

Einer vorteilhaften Ausgestaltung des Erfindungsgedankens zufolge ist vorgesehen, dass der Extremitätenabschnitt außer der Funktionsnaht keine weitere Naht aufweist, die sich entlang des Extremitätenabschnitts von dem Rumpfbereich zu dem Endbereich des Extremitätenabschnitts erstreckt. Es hat sich gezeigt, dass entlang der einen Funktionsnaht für viele Anwendungsfälle ausreichende Kräfte und Momente erzeugt werden können, um die gewünschte therapeutische Wirkung zu erzeugen. Zusätzliche Nähte, die mit einem erhöhten Herstellungsaufwand und einem gegebenenfalls im Bereich der Naht reduzierten Tragekomfort verbunden sind, können zweckmäßig sein, wenn an unterschiedlichen Bereichen zusätzlich zu der üblichen Kompressionswirkung unterschiedlich starke oder verschieden gerichtete Kräfte oder Momente gewünscht sind.

In besonders einfacher Weise kann die unterschiedliche Vorspannung der Randbereiche entlang der Funktionsnaht dadurch bewirkt werden, dass eine erste Nahtlänge eines ersten Randbereichs des elastischen textilen Materials kürzer ist als eine zweite Nahtlänge eines zweiten Randbereichs des elastischen textilen Materials des Extremitätenabschnitts, sodass der erste Randbereich unter einer Vorspannung längs der Funktionsnaht mit dem zweiten Randbereich vernäht ist. Bei einem Verbinden der beiden Randbereiche muss der erste Randbereich des elastischen textilen Materials mit der kürzeren ersten Nahtlänge gedehnt werden, so dass bei einem Verbinden der beiden Randbereiche entlang der Funktionsnaht beide Randbereiche gleichlang sind. Der an den ersten Randbereich des elastischen textilen Materials angrenzende Bereich wird dadurch mit einer Vorspannung versehen, wodurch die Formgebung des Extremitätenabschnitts beeinflusst werden kann und bei der bestimmungsgemäßen Verwendung der Orthese zusätzlich zu der Kompressionswirkung durch die in Umfangsrichtung unterschiedliche Vorspannung weitere Kräfte und Momente auf die betreffende Extremität ausgeübt werden können. Die auf unterschiedliche Art und Weise erzeugbare in Umfangsrichtung unterschiedliche Vorspannung eignet sich besonders zur Erzeugung von Rotationskräften, bzw. Drehmomenten, die einer unerwünschten Verdrehung der Extremität entgegenwirken können oder eine gewünschte Verdrehung der Extremität bei häufigen Bewegungsabläufen unterstützen können.

Die unterschiedliche Vorspannung entlang der Funktionsnaht kann in einfacher Weise während der Herstellung des Extremitätenabschnitts dadurch vorgegeben werden, dass der Materialzuschnitt des Extremitätenabschnitts bezüglich jeder Längsachse des Materialzuschnitts unsymmetrisch ist.

Es hat sich gezeigt, dass zusätzlich zu der Kompressionswirkung eine für viele Anwendungsfälle vorteilhafte therapeutische Wirkung der Orthese dadurch begünstigt werden kann, dass eine sich von dem Rumpfabschnitt bis zu dem Endbereich des Extremitätenabschnitts erstreckende Mittenlinie des Extremitätenabschnitts einen gekrümmten Verlauf aufweist. Wird der gekrümmte Extremitätenabschnitt der Orthese von einem Benutzer verwendet, indem der Benutzer beispielsweise einen Arm oder ein Bein in den gekrümmten Extremitätenabschnitt einführt, wird der Extremitätenabschnitt verformt und liegt eng an dem Arm oder Bein des Benutzers an. Die Verformung des zunächst gekrümmten Extremitätenabschnitts aus dem elastischen textilen Material erzeugt Rückstellkräfte, die zusätzlich zu der Kompressionswirkung während der Benutzung der Orthese weitere Kräfte oder Drehmomente auf den Arm oder das Bein des Benutzers ausüben.

Nachfolgend werden einige exemplarische Ausführungsbeispiele einer erfindungsgemäßen Ausgestaltung einer Orthese beschrieben, die in der Zeichnung dargestellt sind. Es zeigt:
Fig. 1 eine Orthese für einen Oberkörper aus einem elastischen textilen Material,
Fig. 2 einen Ärmel der in Fig. 1 gezeigten Orthese,
Fig. 3 den in Fig. 2 gezeigten Ärmel in einem noch nicht fertig hergestellten Zustand während der Herstellung einer Funktionsnaht,
Fig. 4 eine schematische Darstellung eines Materialzuschnitts, aus welchem ein Ärmel einer erfindungsgemäßen Orthese hergestellt werden kann,
Fig. 5 eine schematische Darstellung eines anderen Materialzuschnitts, aus welchem ebenfalls ein Ärmel einer erfindungsgemäßen Orthese hergestellt werden kann, und
Fig. 6 eine schematische Darstellung einer Orthese für einen Unterkörper.

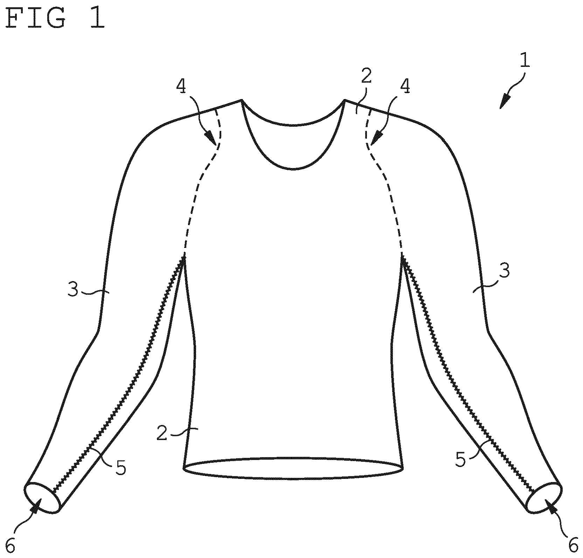

Eine in Fig. 1 schematisch dargestellte Orthese 1 für einen Oberkörper eines Benutzers weist einen Rumpfabschnitt 2 sowie zwei als langarmige Ärmel ausgebildete Extremitätenabschnitte 3 auf. Die Extremitätenabschnitte 3 sind in einem Schulterbereich 4 mit dem Rumpfabschnitt 2 verbunden. Sowohl der Rumpfabschnitt 2 als auch die Extremitätenabschnitte 3 sind jeweils aus einem geeigneten dehnfähigen textilen Material hergestellt, beispielsweise aus hochelastischem Elastan oder aus einem anderen dehnfähigen Kunststofffasermaterial. Die Abmessungen der Orthese 1 werden üblicherweise in Abhängigkeit von dem vorgesehenen Benutzer so vorgegeben, dass die Orthese 1 eng an dem Oberkörper des Benutzers anliegt und kontinuierlich eine Kompressionswirkung auf vorgegebenen Bereiche oder auf den ganzen Oberkörper mit den Armen ausübt.

Die beiden Ärmel, bzw. Extremitätenabschnitte 3 weisen jeweils eine Funktionsnaht 5 auf, die sich von dem Rumpfabschnitt 2 bis zu einem dem Rumpfabschnitt 2 entgegengesetzten Endbereich 6 des Extremitätenabschnitts 3 erstrecken. Der dem Rumpfabschnitt 2 entgegengesetzte Endbereich 6 bildet ein offenes Ende des Ärmels, so dass bei einer bestimmungsgemäßen Verwendung ein Arm des Benutzers von dem Extremitätenabschnitt 3 im Wesentlichen vollständig umhüllt ist, während seine Hand aus dem Extremitätenabschnitt 3 herausragt.

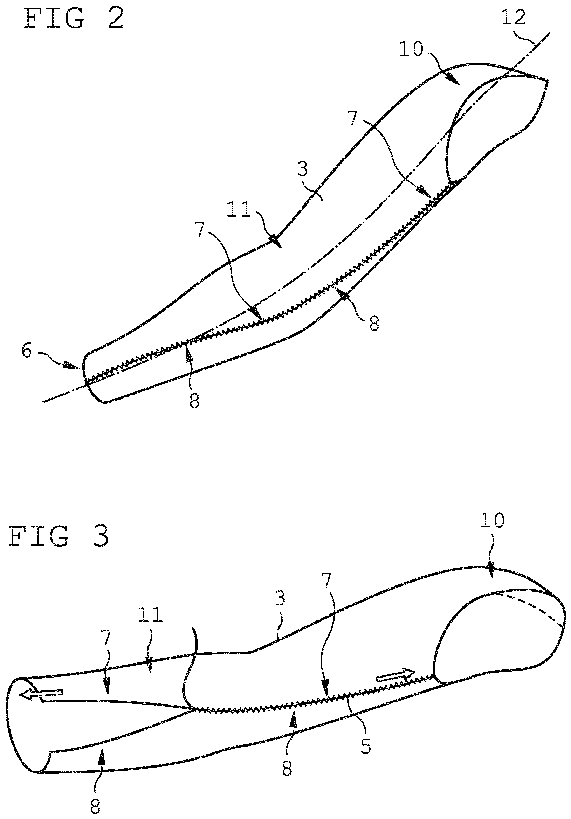

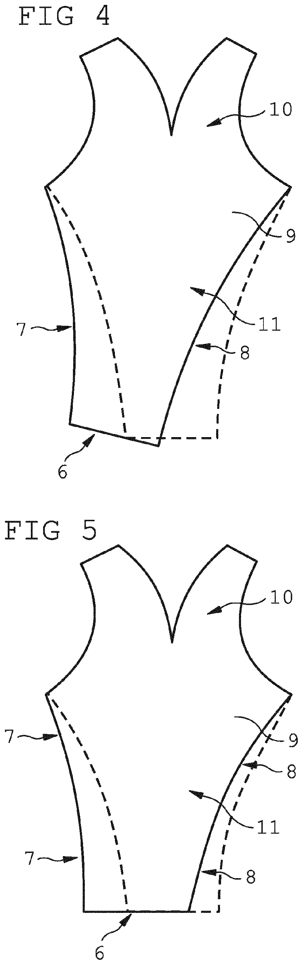

In den Fig. 2 und 3 ist jeweils ein Ärmel der Orthese 1 ohne den Rumpfabschnitt 2 dargestellt, wobei in Fig. 2 der fertig hergestellte Ärmel und in Fig. 3 der Ärmel während der Herstellung, bzw. während des Verbindens von zwei einander zugeordneten Randbereichen 7 und 8 eines einzigen Materialzuschnitts 9 aus einem elastischen textilen Material gezeigt ist. Geeignete Materialzuschnitte 9 sind beispielhaft und schematisch in den Fig. 4 und 5 dargestellt.

Der Materialzuschnitt 9 für den Extremitätenabschnitt 3 weist in einem Übergangsbereich zu dem Rumpfabschnitt 2 einen Schulterabschnitt 10 auf, der in einen Armabschnitt 11 übergeht. Der Armabschnitt 11 weist in Längsrichtung von dem Schulterabschnitt 10 bis zu dem dem Rumpfabschnitt 2 gegenüberliegenden Endbereich 6 verlaufend einen ersten Randbereich 7 und einen gegenüberliegenden zweiten Randbereich 8 auf. Nach dem Verbinden der beiden Randbereiche 7 und 8 zu der Funktionsnaht 5 wird aus dem Materialzuschnitt 9 der Ärmel gebildet. Eine erste Nahtlänge entlang des ersten Randbereichs 7 ist dabei kürzer als eine zweite Nahtlänge entlang des zweiten Randbereichs 8. Um die beiden Randbereiche 7 und 8 so miteinander verbinden zu können, dass die jeweiligen Enden der beiden Randbereiche 7 und 8 an dem Rumpfabschnitt 2 und an dem gegenüberliegenden Endbereich 6 bündig anschließen, muss der erste Randbereich 7 des Materialzuschnitts 9 gedehnt und unter Vorspannung mit dem zweiten Randbereich 8 zu der Funktionsnaht 5 verbunden werden.

Bedingt durch die dadurch erzeugte unterschiedliche Vorspannung der beiden Randbereiche 7 und 8 und in Abhängigkeit von dem Verlauf der durch das Verbinden der beiden Randbereiche 7 und 8 erzeugten Funktionsnaht 5 weist der fertig hergestellte Ärmel bzw. Extremitätenabschnitt 3 in Längsrichtung eine gekrümmte Formgebung bzw. eine gekrümmt verlaufende Mittenlinie 12 auf und erzeugt bei einer bestimmungsgemäßen Benutzung eine vorteilhafte Rotationswirkung auf den von dem Extremitätenabschnitt 3 umhüllten Arm des Benutzers. Gesonderte Zugbänder oder Zügel, bzw. Einsätze oder Aufsätze aus einem anderen Material oder mit abweichend vorgegebenen elastischen Eigenschaften sind nicht erforderlich.

Bei dem in Fig. 4 beispielhaft gezeigten Materialzuschnitt 9 ist der Armabschnitt 11 mit den beiden Randbereichen 7 und 8 gegenüber einem üblichen Materialzuschnitt, der in gestrichelten Linien angedeutet ist, mit einem symmetrisch ausgebildeten Armabschnitt verkippt, ohne den Schulterbereich 10 zu verändern. Bei dem in Fig. 5 beispielhaft gezeigten Materialzuschnitt 9 ist der Randbereich 7 von dem Rumpfabschnitt 2 bis zu dem Endbereich 6 nach außen verschoben, während der gegenüberliegende Randbereich 8 in Längsrichtung jeweils etwa um einen gleichgroßen Abstand nach innen verschoben ist, so dass sich der Abstand der beiden Randbereiche 7 und 8 in Umfangsrichtung des Ärmels und damit die vorgesehene Kompressionswirkung auf den Arm nicht wesentlich verändert. Die beiden Randbereiche 7 und 8 und damit auch der Materialzuschnitt 9 sind nicht symmetrisch ausgebildet. Die bei einem Verbinden, beispielsweise Vernähen der beiden Randbereiche 7 und 8 erzeugte Funktionsnaht 5 weist einen gekrümmten bzw. einen in Längsrichtung des Ärmels gewundenen, spiralförmig verdrehten Verlauf auf.

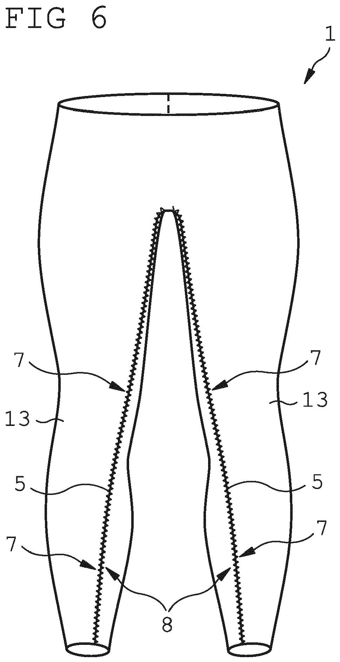

In Fig. 6 ist lediglich beispielhaft eine als Hose ausgestaltete erfindungsgemäße Orthese 1 dargestellt. Zwei lange Hosenbeine 13 weisen ebenfalls eine gekrümmt verlaufende Funktionsnaht 5 auf. Die über die Funktionsnaht 5 miteinander verbundenen Randbereiche 7 und 8 der Hosenbeine 13 weisen eine unterschiedliche Vorspannung auf und erzeugen bei einer bestimmungsgemäßen Verwendung eine gewünschte Rotationswirkung auf die Beine des Benutzers. Neben einem in Fig. 1 dargestellten langarmigen Shirt oder einer in Fig. 6 gezeigten langen Hose sind auch beliebige andere Varianten einer erfindungsgemäßen Orthese 1 wie beispielsweise ein kurzarmiges Shirt, eine kurze Hose oder ein sowohl die Arme als auch Beine umhüllender Anzug denkbar, bei denen die Orthese 1 mindestens eine Extremität des Benutzers mindestens abschnittsweise umhüllt.

## Patentansprüche

1. Orthese (1) mit einem Rumpfabschnitt (2) und mit mindestens einem Extremitätenabschnitt (3), wobei der Extremitätenabschnitt (3) aus einem elastischen textilen Material hergestellt ist und bei der bestimmungsgemäßen Verwendung eine Körperextremität eines Benutzers von dem Rumpfabschnitt (2) bis zu einem dem Rumpfabschnitt (2) abgewandten Endbereich (6) des Extremitätenabschnitts (3) mindestens abschnittsweise umhüllt und dabei eine Kompressionswirkung ausübt, und wobei der Extremitätenabschnitt (3) eine sich entlang des Extremitätenabschnitts (3) von dem Rumpfabschnitt (2) bis zu dem gegenüberliegenden Endbereich (6) erstreckende Funktionsnaht (5) aufweist, **dadurch gekennzeichnet, dass** die beiden längs der Funktionsnaht (5) miteinander verbundenen Randbereiche (7, 8) aus dem elastischen textilen Material eine voneinander abweichende Vorspannung aufweisen.

2. Orthese (1) nach Anspruch 1, **dadurch gekennzeichnet, dass** der Extremitätenabschnitt (3) aus einem einzigen Materialzuschnitt (9) des elastischen textilen Materials hergestellt ist.

3. Orthese (1) nach Anspruch 1 oder Anspruch 2, **dadurch gekennzeichnet, dass** der Extremitätenabschnitt (3) außer der Funktionsnaht (5) keine weitere Naht aufweist, die sich entlang des Extremitätenabschnitts (3) von dem Rumpfbereich (2) zu dem Endbereich (6) des Extremitätenabschnitts (3) erstreckt.

4. Orthese (1) nach einem der vorangehenden Ansprüche, **dadurch gekennzeichnet, dass** eine erste Nahtlänge eines ersten Randbereichs (7) des elastischen textilen Materials kürzer ist als eine zweite Nahtlänge eines zweiten Randbereichs (8) des elastischen textilen Materials des Extremitätenabschnitts (3), sodass der erste Randbereich (7) unter einer Vorspannung längs der Funktionsnaht (5) mit dem zweiten Randbereich (8) verbunden ist.

5. Orthese (1) nach einem der vorausgehenden Ansprüche, **dadurch gekennzeichnet, dass** der Materialzuschnitt (9) des Extremitätenabschnitts (3) bezüglich jeder Längsachse des Materialzuschnitts (9) unsymmetrisch ist.

6. Orthese (1) nach einem der vorausgehenden Ansprüche, **dadurch gekennzeichnet, dass** eine sich von dem Rumpfabschnitt (2) bis zu dem Endbereich (6) des Extremitätenabschnitts (3) erstreckende Mittenlinie (12) des Extremitätenabschnitts (3) einen gekrümmten Verlauf aufweist.

## Claims

1. Orthosis (1) comprising a trunk portion (2) and with at least one extremity portion (3), wherein the extremity portion (3) is produced from an elastic, textile material and, when used as intended, encloses a body extremity of a user from the trunk portion (2), at least in sections, all the way to an end region (6) of the extremity portion (3) facing away from the trunk portion (2) and thereby exerts a compressive effect, and wherein the extremity portion (3) comprises a functional seam (5) extending alongside the extremity portion (3) from the trunk portion (2) to the opposite end region (6), **characterized in that** the two end regions (7, 8) made of the elastic textile material and connected to one another along the functional seam (5) have pre-stresses that are different from one another.

2. Orthosis (1) according to claim 1, **characterized in that** the extremity portion (3) is produced from a single material blank (9) of the elastic, textile material.

3. Orthosis (1) according to claim 1 or claim 2, **characterized in that** the extremity portion (3) does not comprise any further seam besides the functional seam (5) that extends along the extremity portion (3) from the trunk region (2) to the end region (6) of the extremity portion (3).

4. Orthosis (1) according to one of the preceding claims, **characterized in that** a first seam length of a first edge region (7) of the elastic textile material is shorter than a second seam length of a second edge region (8) of the elastic textile material of the extremity portion (3), so that the first edge region (7), under a pre-stress, is connected to the second edge region (8) along the functional seam (5).

5. Orthosis (1) according to one of the preceding claims, **characterized in that** the material blank (9) of the extremity portion (3) is non-symmetric relative to each longitudinal axis of the material blank (9).

6. Orthosis (1) according to one of the preceding claims, **characterized in that** a center line (12) of the extremity portion (3) extending from the trunk portion (2) to the end region (6) of the extremity portion (3) has a curved course.

## Revendications

1. Orthèse (1) comprenant une section de torse (2) et comprenant au moins une section d'extrémité (3), la section d'extrémité (3) étant fabriquée dans une matière textile élastique et, lors de l'utilisation prévu, entourant au moins par sections une extrémité du corps d'un utilisateur, allant de la section de torse (2) jusqu'à une zone terminale (6) de la section d'extrémité (3), détournée de la section de torse (2), et exerçant ainsi un effet de compression, et la section d'extrémité (3) présentant une couture de fonction (5) s'étendant de la section de torse (2) jusqu'à la zone terminale opposée (6) le long de la section d'extrémité (3), **caractérisée en ce que** les deux parties de bord (7, 8) reliées entre elles le long de la couture de fonction (5), constituées de la matière textile élastique, présentent une pré-tension différente l'une de l'autre.

2. Orthèse (1) selon la revendication 1, **caractérisée en ce que** la section d'extrémité (3) est fabriquée dans une seule pièce découpée de matière (9) de la matière textile élastique.

3. Orthèse (1) selon la revendication 1 ou la revendication 2, **caractérisée en ce que** la section d'extrémité (3), sauf la couture de fonction (5), ne présente pas d'autre couture qui s'étende le long de la section d'extrémité (3), depuis la section de torse (2) jusqu'à la zone terminale (6) de la section d'extrémité (3).

4. Orthèse (1) selon l'une quelconque des revendications précédentes, **caractérisée en ce qu'**une première longueur de couture d'une première zone de bord (7) de la matière textile élastique est plus courte qu'une deuxième longueur de couture d'une deuxième zone de bord (8) de la matière textile élastique de la section d'extrémité (3), de sorte que la première zone de bord (7) est reliée, sous pré-tension, à la deuxième zone de bord (8) le long de la couture de fonction (5).

5. Orthèse (1) selon l'une quelconque des revendications précédentes, **caractérisée en ce que** la pièce découpée de matière (9) de la section d'extrémité (3) est asymétrique en ce qui concerne chaque axe longitudinal de la pièce découpée de matière (9).

6. Orthèse (1) selon l'une quelconque des revendications précédentes, **caractérisée en ce qu'**une ligne médiane (12) de la section d'extrémité (3), s'étendant de la section de torse (2) jusqu'à la zone terminale (6) de la section d'extrémité (3), présente une allure courbée.
